# EUROPEAN PATENT APPLICATION

(11) **EP 0 911 056 A1**
(43) Date of publication of application: **28.04.1999**
(21) Application number: 98308602.6
(22) Date of filing: 21.10.1998
(51) Int. Cl.: A61M 35/00, A61F 9/00

(54) **Ocular application device**

(30) Priority: 21.10.1997 GB 9722145
(71) Applicant: THE MENTHOLATUM COMPANY LIMITED, Peel Park Campus, East Kilbride G74 5PE (GB)
(72) Inventor: Hobbs, Michael Anthony, Woodford Green, Essex IG8 0LD (GB); Calvert, John Richard, Epping, Essex CM16 4LS (GB)
(74) Representative: McNally, Roisin

(57) **Abstract**

Eye spray devices with a baffle to reduce the force from the spray, and/or a spacer to space a spray head from a user's eye. A spray device can have a housing holding a spray container where the spray is activated by a manual pump head and the housing includes a mesh baffle. The whole unit may be sealed to remain sterile until it is used.

## Description

This invention relates to an ocular application device capable of efficient and comfortable dosage to the eye.

There are many common eye complaints and diseases associated with eyes which require preparations and treatments to be administered directly to the eye.

One category of common eye irritation occurs when there are particulates in the atmosphere. These particulates can be in the form of pollen, dust or grit particles or cigarette smoke.

A second category of eye irritation is caused by the quality of the air around the eye. In most major conurbations, the extensive use of the automobile causes pollutants to be released into the atmosphere. This problem can be exacerbated by industrial pollution. Although these pollutants are invisible they can still be a source of irritation for eyes. As examples, oxides of nitrogen (produced by industry and un-catalysed automobile exhausts) and oxides of sulphur (predominantly produced by industry and some fossil fuel burning power stations) can cause eye irritation.

Similarly, chemicals found in swimming pools can cause eye irritation.

Other external influences can cause eye irritation. Exposure of eyes to artificial light; either from working with a VDU or from glare, such as the glare from cars' headlights when driving at night, can cause squinting and/or tear production. The use of contact lenses, especially when left in the eye for longer than directed, can cause severe eye irritation, again in the form of tear production or causing eyes to become blood shot.

There are several remedies available on the market to combat eye irritation. Eye protection in the form of goggles, especially for those working in dusty environments, will of course help to prevent eye irritation by eliminating the exposure of the eye to dust and/or grit particles. This protection can be in the form of devices ranging from simple goggles, which merely cover the eyes, to more complicated face masks which can have their own air supply.

As well as devices which attempt to prevent the eye irritation from occurring, eye irritation cures are available. The irritation relieving preparations are commonly in an aqueous medium and are applied to the eyes with the use of squeezable bottles or other devices such as eye droppers.

Examples of preparations which may be applied directly to an eye include ocular anti-infectives, ocular antiinflammatories, treatments for glaucoma, ocular lubricants, mydriatics, anaesthetics, ocular stains and anti-allergic drugs.

Most of the eye preparation application devices have at least one drawback. With squeezable bottles and eye dropping devices, not only is it difficult to precisely apply the correct amount of preparation to the eye but also after the squeezable bottle or the eye dropping solution has been used once, the previously sterile solution may become contaminated with an airborne microbe. Thus, contamination of the preparation may affect its irritant relieving properties.

Use of spray nozzle application devices can solve both of these problems. Each operation of a spray can be made to deliver a measured amount of preparation. However, it is difficult for the user to know the correct distance that the nozzle should be held away from the eye when applying the preparation. Also it can be difficult for the user to keep their eyes open when facing the spray.

It is an objective of the present invention to provide an improvement to eye spray devices and to provide a device capable of efficient and comfortable dosage of treatments to the eye.

In one aspect the present invention provides a baffle for an eye spray device such that spray is directed away from the pupil when a user looks directly towards the device.

A baffle positioned between a spray device and the pupil of the user protects the most delicate central portion of the eye whilst allowing spraying.

The baffle may form part of a spray nozzle.

Alternatively the baffle may form part of an eye spray device.

Alternatively the baffle may form part of a spacer device as described herein.

Preferably the baffle is of round configuration.

In use the baffle prohibits a direct path of spray to the most sensitive area of the eye whilst causing turbulence and thereby providing a gentler low velocity misting action.

According to a second aspect of the present invention there is a spacer device which can be applied to an eye spray container such that the device enables an eye spray to be held at a fixed distance from the user's eyes.

In one embodiment the spacer device can be connectable to a spray container and extendible therefrom.

Alternatively the spacer can be integral to a spray container.

Preferably the spacer device forms an elliptical shape which can be held against a user's eye socket.

In a particularly preferred embodiment the spacer includes a baffle as described herein.

In a particular embodiment the invention provides a housing for holding a spray container wherein the housing comprises a spacer device as described herein.

The housing can further comprise a baffle.

Preferably the housing comprises two co-operating portions wherein the lower portion supports a spray container and the upper portion is moveable between a protecting position and a spraying position.

In one embodiment the upper portion pivots between positions and is restricted in motion by interaction with the lower portion.

In an alternative embodiment the upper portion is moveable in line with the housing.

Preferably the spacer device, baffle, housing and spray container are of lightweight, rigid, plastic construction.

In a particularly preferred embodiment the baffle comprises a mesh.

The mesh baffle reduces the speed and ferocity of the spray as it hits the eye. A number of fine mesh materials made of a variety of different plastics may be used. The mesh is placed between the spray nozzle and the eye and reduces a rapidly moving spray to a fine, gentle mist which is very easy to apply to the surface of the eye. The mesh has the added advantage that the user cannot see the spray head when they pump the actuator.

Because the mist is so much finer, it is possible to place the eye quite close to the mesh and there is no need for an eye cup to locate the position of the device in relation to the users eye.

In a preferred embodiment the spray head is inside a sealed, sterile unit. This means that excessive liquid from the spray head is held on the inside of the mesh and runs back into the liquid reservoir. This minimises any waste as the only liquid escaping from the unit is a fine mist which is being directed towards the eye. The spray head is activated via a manual pump head which extends above the spray actuator. The complete unit is sealed using special 'o' rings. The whole unit can be sterilised after assembly and the mesh will be closed with a plastic cap. Thus the whole unit remains sterile and tamper evident until the tamper ring is removed by the consumer. It is only at this point that the product becomes "live" and the preservative system within the solution will maintain the sterility of the liquid for 1-2 months while the product is being used.

Preferably the fine mesh is moulded into the body of the unit. It is made of a very strong plastic material with holes so fine that the liquid inside the reservoir cannot leak out. However, the fine atomised particles from the spray head are able to penetrate the mesh thus achieving the fine mist in normal use.

The invention will now be further described with reference to the accompanying drawings in which:

Figure 1 shows a front elevation of an eye spray device according to the invention.

Figure 2 shows a sectioned side view of an eye to which eye irrigation is being applied via a spray.

Figure 3 shows a baffle embodiment of labyrinth construction.

Figure 4 shows a baffle embodiment of mesh construction.

Figure 5 shows a cross sectional view of a baffle.

Figure 6 shows an alternative cross sectional view of a baffle.

Figure 7 diagrammatically represents views of one embodiment of the device showing a container of eye irritant remedy within a container.

Figure 8 diagrammatically illustrates an alternative embodiment of the device of Figure 7.

Figure 9 diagrammatically illustrates a preferred pump for use with the invention.

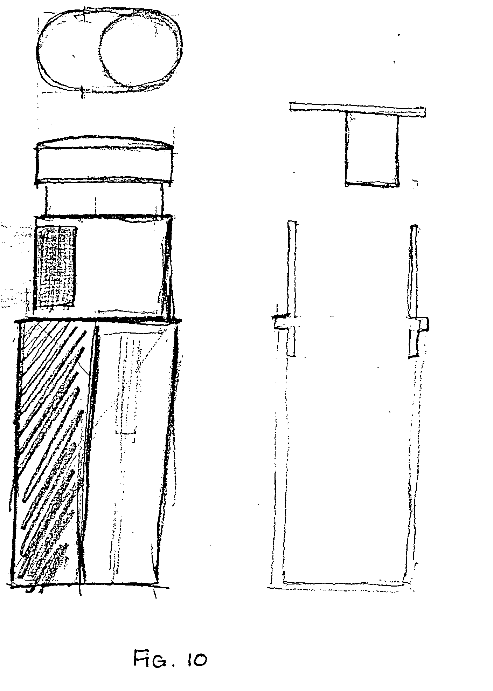

Figure 10 illustrates a spray device of the invention from outside.

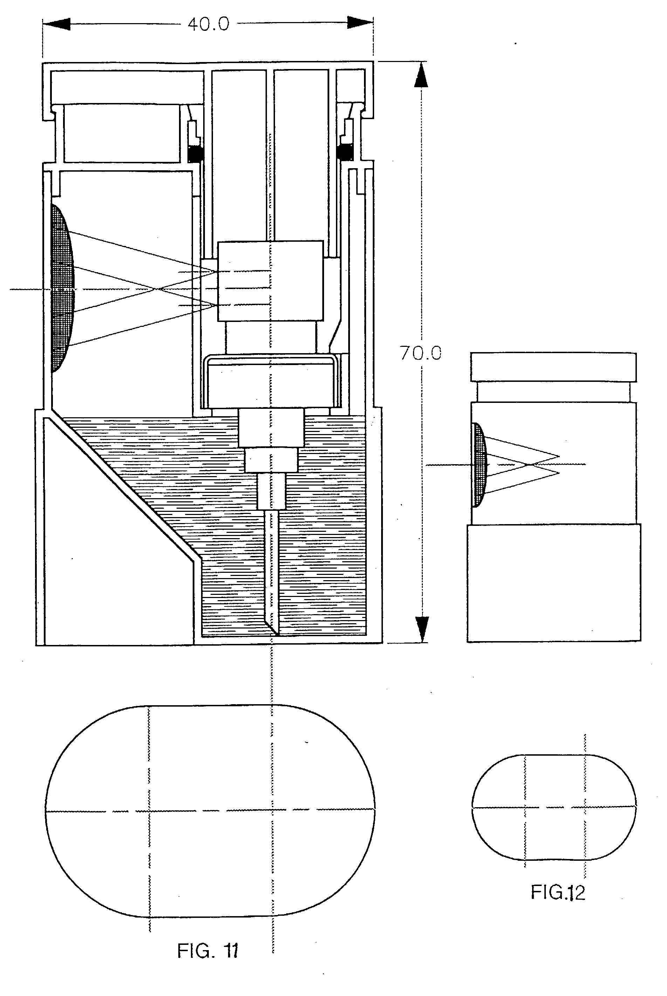

Figure 11 illustrates one spray head activator in a basic container of eliphical shape.

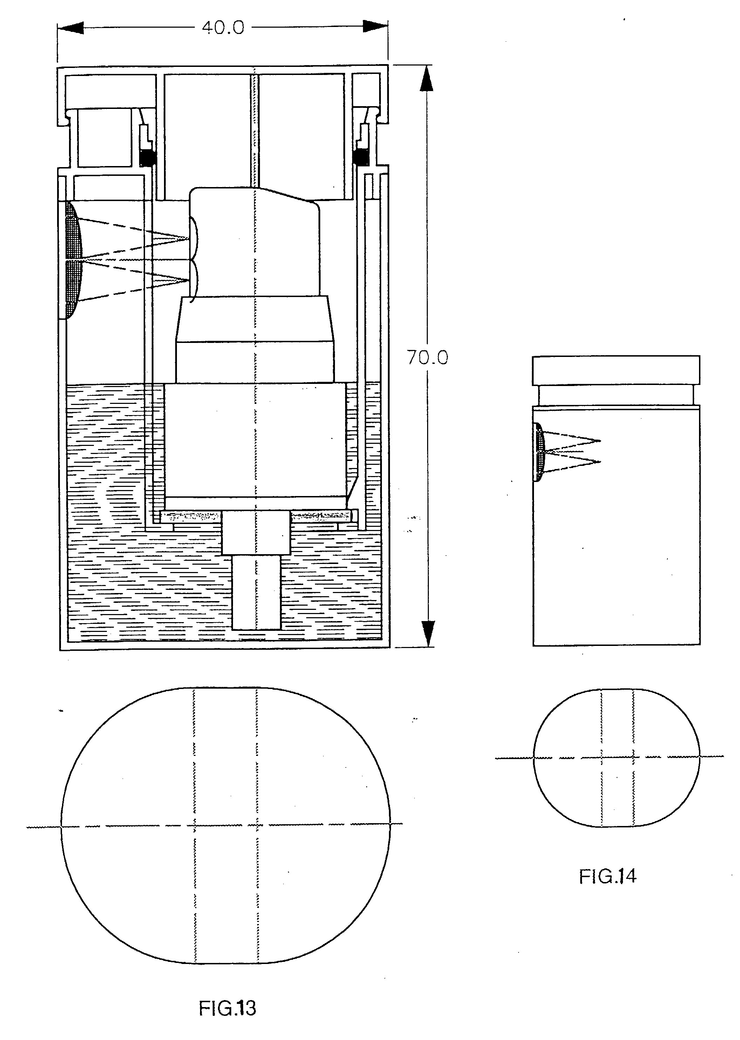

Figure 12 illustrates an actual size of the device of Figure 11.

Figure 13 illustrates an alternative spray head actuator in a basic container of ocular shape.

Figure 14 illustrates an actual size of the device of Figure 13.

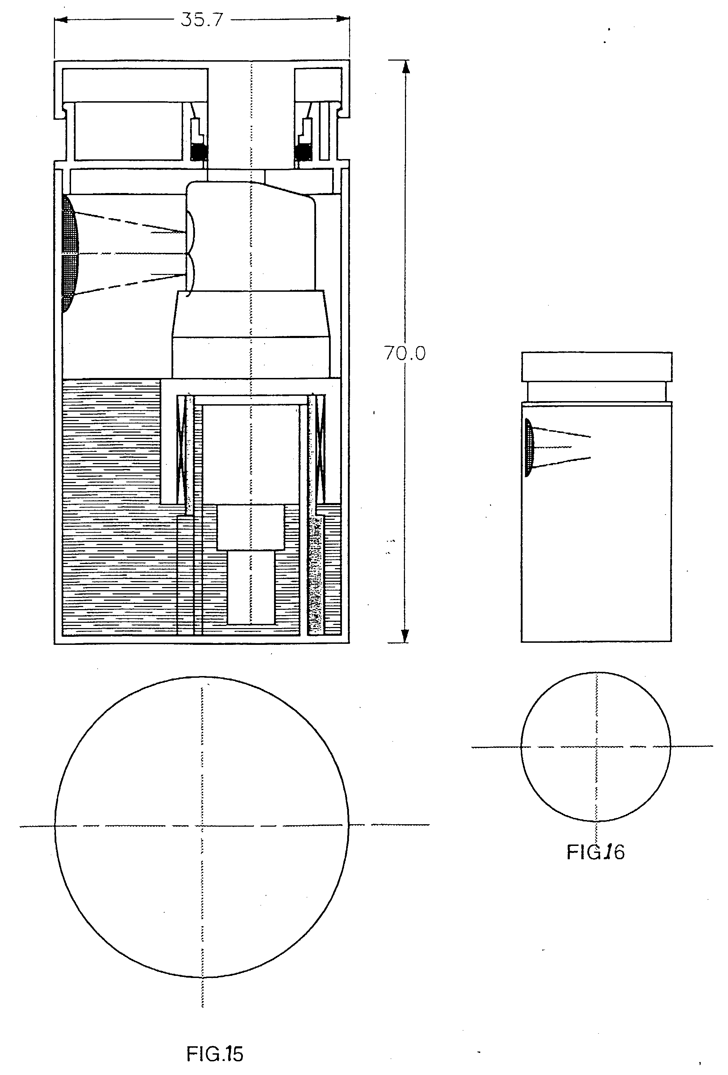

Figure 15 illustrates a spray head actuator in a basic container of circular shape.

Figure 16 illustrates an actual size of the device of Figure 15.

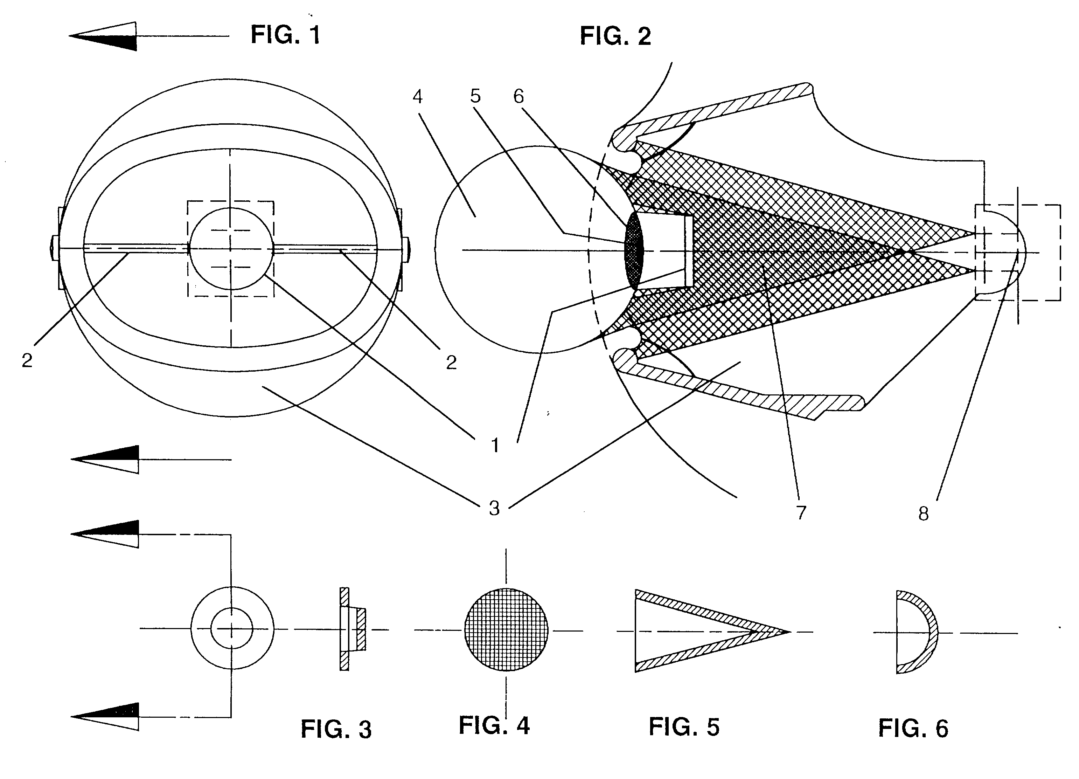

Referring to the drawing, Figure 2 shows a sectioned side view of an eye to which eye irrigation is being applied via a spray 7.

The sprayhead 8 directs a spray pattern towards the eye 4.

The outer eye piece structure 3 acts as a spacer and typically locates around the eye 4 thereby distancing the eye 4 from the spray head 8.

Attached to the outer eye piece spacing structure 3, either integrally or separately, is a baffle 1.

The baffle 1 is located, or attached, by means of at least one supporting member 2.

The baffle, when not an integral part of the outer eye piece structure, may be so constructed as to be removable.

In Figure 3 the baffle is of labyrinth construction and in Figure 4 the baffle is of mesh construction. Either construction will assist misting of the spray.

A baffle may be designed as part of the nozzle of a spray container.

In Figure 5 the baffle is of conical form whilst in Figure 6 the baffle is of hemispherical form.

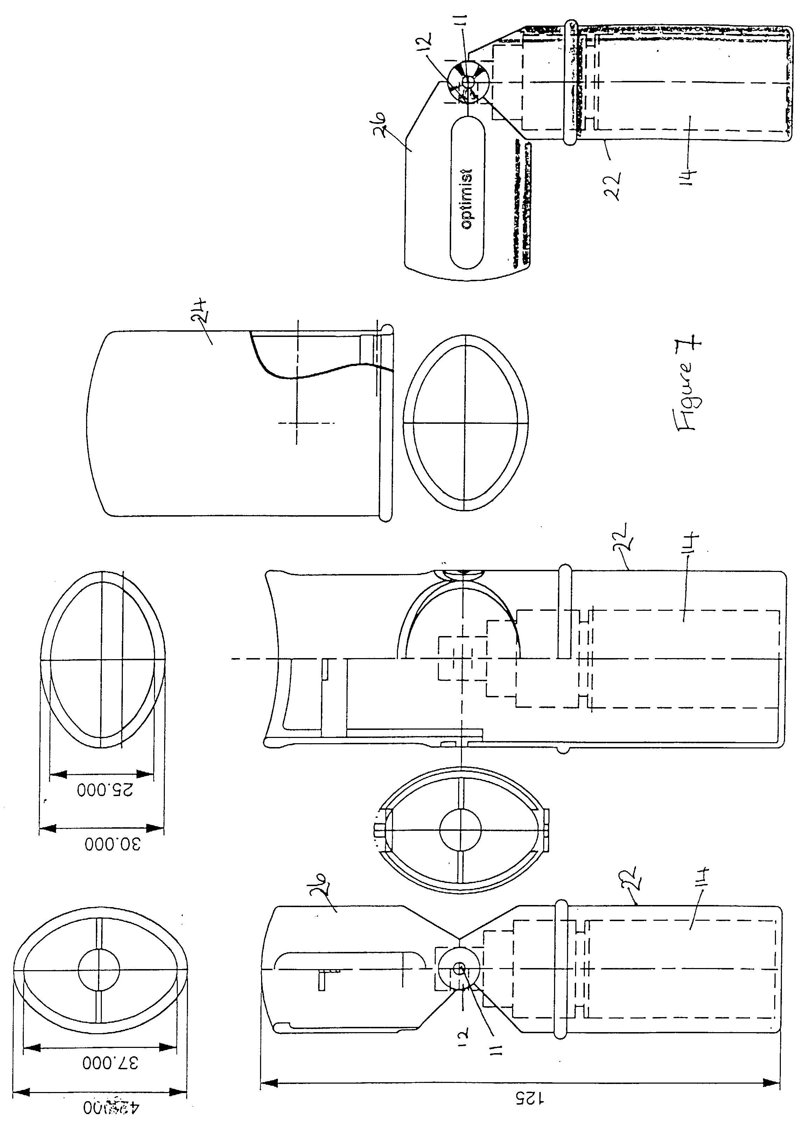

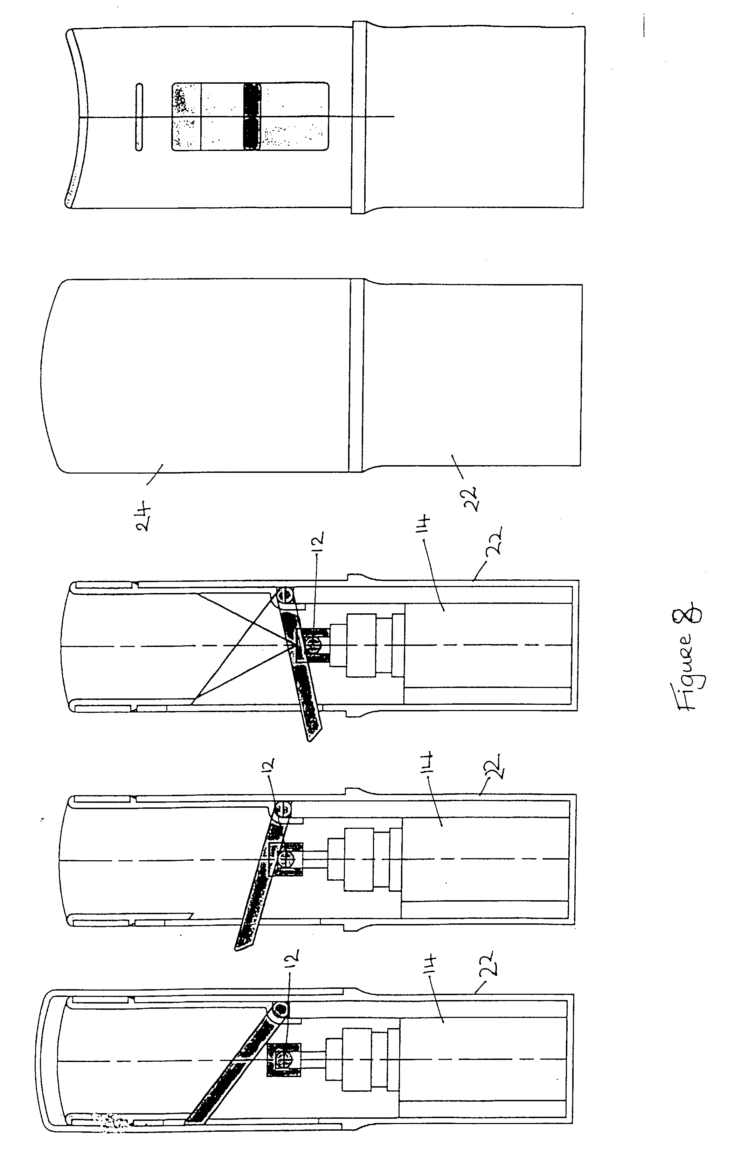

As can be seen in Figures 7 and 8 an eye spray device according to the invention can comprise a housing 22 into which a spray container 14 can be fitted. The spray container may or may not be an exchangeable unit.

In the embodiment shown in Figure 7 the housing comprises a moveable portion 26 which acts as a spacer such that when spray is emitted from the nozzle 12 of container 14 the spacer 26 can be held against the user's eye socket to ensure that the spray is directed at the eye whilst being held at a fixed distance.

In the embodiment of Figure 7 the moveable part 26 is telescopically arranged in line with the housing

The spray can be engineered to deliver a measured amount of dose solution per activation.

In Figure 7 the spacer 26 can be attached to the housing at pivot point 11 and when the device is not being used the spacer can be placed upright for storage and carrying.

The devices depicted in Figures 7 and 8 each include protective covers 24.

In an alternative embodiment the spacer may simply comprise an attachment as in Figure 1 whereby it can be directly applied to the neck of a spray container 14 and may be of less rigid construction.

The housing 22 may be constructed of a material which will allow it to be sterilized by microwave.

A device as shown in Figures 7 and 8 is portable and preferably pocket size.

To prevent spray being aimed directly at the user's iris and pupil it is preferable that the spray nozzle contains a baffle as depicted in Figures 1, 3 or 4 to ensure spray is directed away from the centre of the eye.

Alternatively, the spacer can contain a baffle as depicted in Figures 1, 3 or 4 at any position between the nozzle spray and the user's eye to prevent spray being directed at the iris or pupil of the user.

This arrangement ensures that the user cannot look directly at the spray nozzle during use.

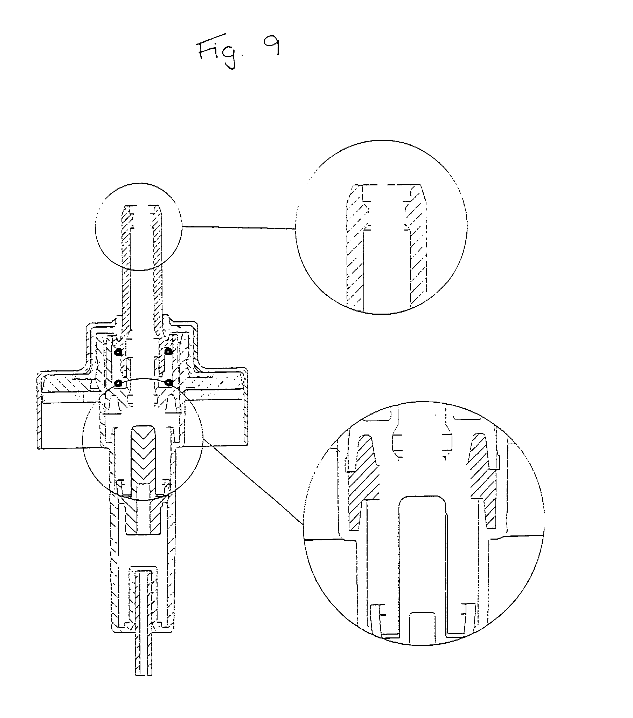

Suitably a device as depicted in Figures 7 and 8 can employ a spray container using a pharmaceutical pump and nozzle arrangement. A particularly preferred pump is the type shown in Figure 9, known as the S4 Pump of Spruhventile/Somova. This pump enables consistent spray delivery independent of finger pressure.

Delivery is achieved by a double action mechanism which actuates the spray only when a predetermined internal pressure is reached. This avoids problems of partial or double doses.

The pump enables extremely accurate dosage and is top crimp-sealed which gives protection against evaporation and prevents extraneous air entering the product, minimising contamination.

## Claims

1. A spray device suitable for delivering preparations to a user's eye, the device including a baffle such that the spray does not directly hit a user's pupil.

2. A spray device suitable for delivery preparation to a user's eye, the device comprising a spacer which enables a spray nozzle to be held at a fixed difference from a user's eye.

3. A baffle device for attachment to a spray device.

4. A spacer device for attachment to a spray device.

5. A spray device as claimed in claim 1 or claim 2 comprising a baffle and/or a spacer.

6. A housing for holding an eye spray device wherein the housing comprises a spacer device and/or a baffle.

7. A housing as claimed in claim 6 comprising two co-operating portions wherein the lower portion supports a spray container and the upper portion is movable between a protecting position and a spraying position.

8. A baffle device as claimed in claim 3 comprising a mesh.

9. A spray device as claimed in any of claims 1, 2 and 5 comprising a spray container in a housing whereby the spray head is activated via a manual pump head which extends above the spray actuator and the housing wherein the housing comprises a baffle of fine mesh material moulded into a housing body.
